# EUROPEAN PATENT SPECIFICATION

(11) **EP 1 499 372 B1**
(45) Date of publication and mention of the grant of the patent: **28.12.2011**
(21) Application number: 03726494.2
(22) Date of filing: 28.04.2003
(51) Int. Cl.: A61M 5/14, G06F 19/00

(54) **MEDICAL TREATMENT VERIFICATION METHOD**
VERFAHREN ZUR ÜBERPRÜFUNG MEDIZINISCHER BEHANDLUNGEN
PROCEDE DE VERIFICATION D'UN TRAITEMENT MEDICAL

(30) Priority: 30.04.2002 US 135180
(43) Date of publication of application: 26.01.2005
(73) Proprietor: Baxter International Inc., Deerfield, Illinois 60015 (US)
(72) Inventor: BUI, Tuan, Green Oaks, IL 60048 (US); ACHARYA, Meetali, Burlington, Ontario L7P 1P2 (CA); WILKES, Gordon, Newmarket, Ontario L3Y 8B9 (CA); MARTUCCI, James, Libertyville, IL 60048 (US); PAUL, Eric, North York, Ontario M3H 5Z5 (CA); MIHAI, Dan, Hanover Park, IL 60133 (US)
(74) Representative: Probert, Gareth David
(86) International application number: PCT/US2003/013110
(87) International publication number: WO 2003/092769

(56) References cited:
- EP-A2- 0 960 627
- US-A- 5 781 442
- US-A- 5 788 669
- US-A- 6 135 949
- US-A1- 2002 038 392

## Description

### Technical Field

This invention relates generally to a method for operating medical devices and communication between such devices. More particularly, the present invention relates to a method for verifying that the right medication is provided to the right patient in the right dose at the right time, and via the right route.

### Background of the Invention

Patient care systems typically include computer networks, medical devices for treating a patient, and controls for the medical devices. Although patient care systems have been improved through the use of computerized automation systems and methods, patient care systems continue to reply heavily upon manual data management processes for medical devices and controls for medical devices. For example, musing stations are typically connected to the computer networks in modern hospitals, but it is unusual for the computer network to extend to a patient's room. Computer network offer the opportunity for automated data management processing including the operating and monitoring of medical devices and controls for the medical devices at the point-of-care. Despite advances in the field, automated data management technology has been underutilized for point-of-care applications due to a lack of more efficient systems and methods for operating medical devices such as infusion pumps.

### Summary of the Invention

In accordance with the present invention, there is provided a method according to claims 1 and 11.

The present invention provides a method for operating medical devices. The method may be used to program an infusion pump. The system may be implemented in a variety of ways including as a computer program. When implemented as a computer program, the program includes logic for: accessing information related to the identity of a patient; accessing information regarding the identity of a medical treatment, the medical treatment having a treatment type; determining whether the medical treatment has been previously associated with the patient; providing a first error signal if the medical treatment has not been previously identified with the patient; accessing information related to the identity of a medical device, the medical device configured to administer the medical treatment type; determining whether a plurality of operating parameters for the medical device are consistent with the medical treatment, the operating parameters having been provided from a central computer, the operating parameters having been provided to the medical device without passing through a remote computer; providing a second error signal if the operating parameters for the medical device are not consistent with the medical treatment.

Other advantages of the present invention will be, or will become, apparent to one having ordinary skill in the art upon examination of the following drawings and detailed description.

### Brief Description of the Drawings

The invention can be better understood with reference to the following drawings. The components in the drawings are not necessarily to scale, emphasis instead being placed upon clearly illustrating the principles of the present invention. In the drawings, like reference numerals designate corresponding parts throughout the several views.
FIG. 1 is a graphical representation of a patient care system. The patient care system includes a pharmacy computer, a central system, and a digital assistant at a treatment location.
FIG. 2 is a block diagram of a computer system that may be representative of the pharmacy computer, the central system, and/or the digital assistant of FIG. 1. The computer system includes a medical treatment verification system or a portion of the medical treatment verification system.
FIG. 3 is a block diagram showing functional components of the patient care system of FIG. 1.
FIG. 4 is a flowchart showing a first exemplar embodiment of the medical treatment verification system of FIG. 2.
FIG. 5 is a flowchart showing a second exemplar embodiment 500 of the medical treatment verification system 210 of FIG. 2.
FIG. 6 is a flowchart showing an example 600 of the medical, treatment verification system 210 of FIG. 2.
FIG. 7 is a flowchart showing an example 700 of the medical treatment verification system 210 of FIG. 2.
FIG. 8 is an exemplar computer screen that is useful in implementing various functions of the patient care system of FIG. 1

### Detailed Description

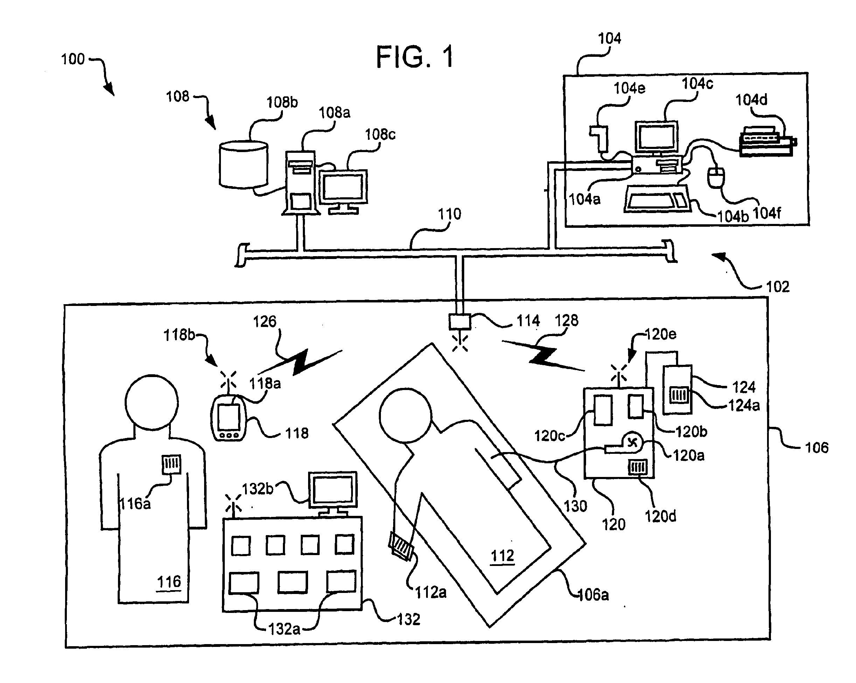

FIG. 1 is a graphical representation of a patient care system 100. The patient care system 100 includes a pharmacy computer 104, a central system 108, and a treatment location 106, linked by a network 102. The pharmacy computer 104 may include a processing unit 104a, a keyboard 104b, a video display 104c, a printer 104d, a bar code reader 104e, and a mouse 104f. Although not shown in FIG. 1, the patient care system 100 may also include subsystems for hospital administration, nursing stations, a clinical information subsystem, a hospital information subsystem, an Admissions Discharge and Transfer (ADT) subsystem, a billing subsystem, and/or other subsystems typically included in patient care systems.

The central system 108 may include a central servicing unit 108a, a database 108b, a video display 108c, input/output components, and many other components known to those having ordinary skill in the art. The network 102 includes a cable communication system 110 portion and a wireless communication system portion. The cable communication system 110 may be, but is not limited to, an Ethernet cabling system, and a thin net system.

The treatment location 106 may include a treatment bed 106a, an infusion pump 120, and medical treatment cart 132. In FIG. 1, a clinician 116 and a patient 112 are shown in the treatment location 106. Medication 124 may be of a type that may be administered using an infusion pump 120. Medication 124 may also be of a type that is administered without using an infusion pump. The medication may be stored in medication storage areas 132a of medical treatment cart 132. The clinician 116 uses a digital assistant 118 to administer medication 124 to the patient 112.

In the course of treating patient 112, the clinician 116 may use the digital assistant 118 to communicate with the cable communication system 110 of the network 102 via a first wireless communication path 126. The infusion pump 120 may also have the ability to communicate with the cable communication system 110 via a second wireless communication path 128. The medication cart 124 may also have the ability to communicate via a wireless communication path (not shown in FIG. 1). A wireless transceiver 114 interfaces with the cable communication system 110. The wireless communication system portion of the network may employ technology such as, but not limited to, that known to those having ordinary skill in the art as IEEE 802.11b "Wireless Ethernet," a local area network, wireless local area networks, a network having a tree topography, a network having a ring topography, wireless internet point of presence systems, an Ethernet, the Internet, radio communications, infrared, fiber optic, and telephone. Though shown in FIG. 1 as a wireless communication system, communication paths may be hardwired communication paths.

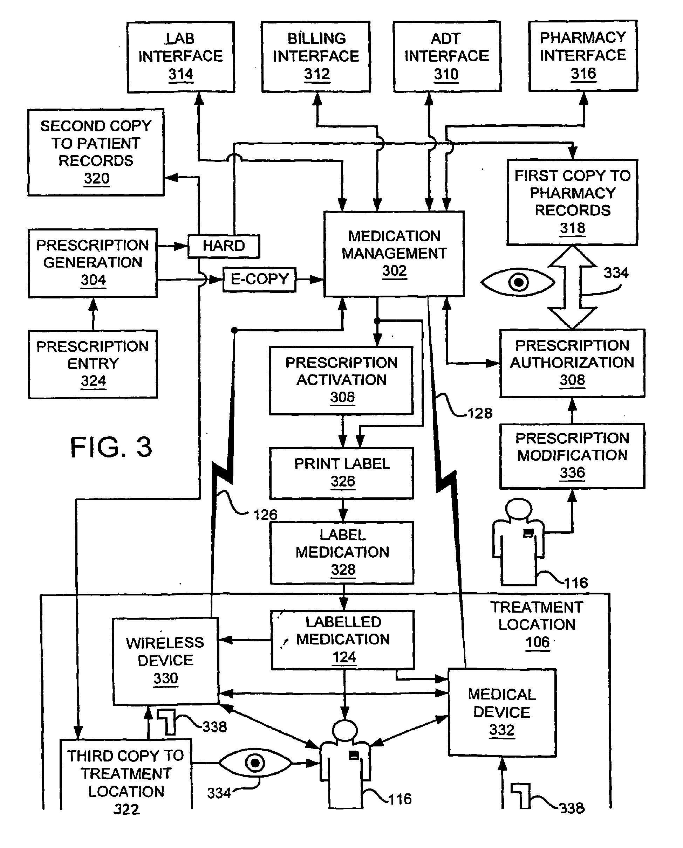

In the patient care system 100, a physician (not shown) may order medication 124 for patient 112. The order may also originate with a clinician 116 at the treatment location 106. The physician and/or clinician 116 may use a computerized physician order entry system (CPOE) and/or the medical cart 132 to order the medication 124 for the patient 112. Those having ordinary skill in the art are familiar with basic CPOEs. Despite its name, any clinician 116 may use the CPOE. If the medication 124 is one that is efficient to administer through infusion pump 120, the order includes information for generating operating parameters for the infusion pump 120. The operating parameters are the information and/or instruction set that is necessary to program a medical device 332 (FIG. 3) to operate in accordance with the order.

The order may be entered in the pharmacy computer 104 via input/output devices such as the keyboard 104b, the mouse 104f, a touch screen display, the CPOE system and/or the medical treatment cart 132. Those having ordinary skill in the art are familiar with these and similar input/output devices. The processing unit 104a is able to transform a manually entered order into computer readable data. Devices such as the computerized prescribing order entry system may transform an order into computer readable data prior to introduction to the processing unit 104a. The operating parameters may then be printed in a bar code format by the printer 104d on a medication label 124a. The medication label 124a may then be affixed to a medication 124 container. The medication 124 container is then transported to the treatment location 106. The medication 124 may then be administered to the patient 112 in a variety of ways known in the art including orally and through an infusion pump 120. If the medication 124 is administered orally, the clinician 116 may communicate via the digital assistant 118 and/or the medical cart 132. The medical cart 132 is computerized and generally has a keyboard (not shown), a display 132b, and other input/output devices such as a bar code scanner (not shown).

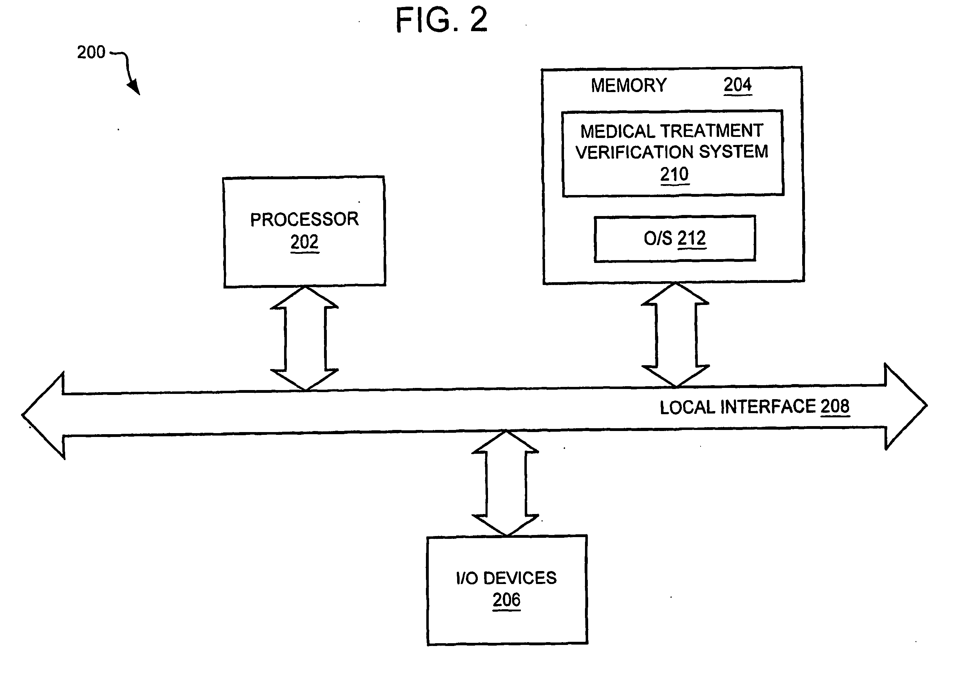

At the treatment location, the medication 124 may be mounted on the infusion pump 120 and an intravenous (IV) line 130 may be run from the infusion pump 120 to the patient 112. The infusion pump 120 may include a pumping unit 120a, a keypad 120b, a display 120c, an infusion pump ID 120d, and an antenna 120e. Prior art infusion pumps may be provided with a wireless adaptor (not shown) in order to fully implement the medical treatment verification system 210 (FIG. 2) of the current invention. The wireless adaptor may have its own battery if necessary to avoid reducing the battery life of prior art infusion pumps. The wireless adaptor may also use intelligent data management such as, but not limited to, store and forward data management and data compression to minimize power consumption. The wireless adaptor may also include the ability to communicate with the digital assistant 118 even when the network 102 is not functioning.

The patient care system 100 may include a variety of identifiers such as, but not limited to, personnel, equipment, and medication identifiers. In FIG. 1, the clinician 116 may have a clinician badge 116a identifier, the patient 112 may have a wristband 112a identifier, the infusion pump 120 may have an infusion pump ID 120d identifier, and the medication 124 may have a medication label 124a identifier. Clinician badge 116a, wristband 112a, infusion pump ID 120d, and medication label 124a include information to identify the personnel, equipment, or medication they are associated with. The identifiers may also have additional information. For example, the medication label 124a may include information regarding the intended recipient of the medication 124, operating parameters for infusion pump 120, and information regarding the lot number and expiration of medication 124. The information included in the identifiers may be printed, but is preferably in a device readable format such as, but not limited to, an optical readable device format such as a bar code, a radio frequency (RF) device readable format such as an RFID, an iButton, a smart card, and a laser readable format. The digital assistant 118 may include a display 118a and may have the ability to read the identifiers including biometric information such as a fingerprint.

The wristband 112a is typically placed on the patient 112 as the patient 112 enters a medical care facility. The wristband 112a includes a patient identifier. The patient identifier may include printed information to identify the patient and additional information such as a treating physician's name(s). The patient identifier for patient 112 may include information such as, but not limited to, the patient's name, age, social security number, the patient's blood type, address, allergies, a hospital ID number, and the name of a patient's relative.

FIG. 2 is a block diagram of a computer 200. Computer 200 may be the pharmacy computer 104, the central system 108, a CPOE, the digital assistant 118 of FIG. 1, and/or a computer included in any number of other subsystems that communicate via the network 102. Computer 200 includes a medical treatment verification system 210. In some embodiment, the medical treatment verification system 210 verifies that the right medication is provided to the right patient in the right dose at the right time, and via the right route. In other embodiments, the medical treatment verification system 210 provides a subset of these desirable verification features. In some embodiments, the programming of the infusion pump 120 may be based on operating parameters received from the pharmacy computer 104, and/or another remote computer. In other embodiments, the programming of the infusion pump 120 may be based on operating parameters that are confirmed as correct by the pharmacy computer 104, another remote computer, and/or the clinician 116. The operating parameters and/or confirmations may be transported via the cable communication system 110 and the first and second wireless communication paths 126 and 128.

A critical concern in the art is that the right medication is administered to the right patient. Therefore, the medical treatment verification system 210 includes features to assure the right medication is administered to the right patient in an efficient manner. The medical treatment verification system 210 of the invention can be implemented in software, firmware, hardware, or a combination thereof. In one mode, the medical treatment verification system 210 is implemented in software, as an executable program, and is executed by one or more special or general purpose digital computer(s), such as a personal computer (PC; IBM-compatible, Apple-compatible, or otherwise), personal digital assistant, workstation, minicomputer, or mainframe computer. An example of a general purpose computer that can implement the medical treatment verification system 210 of the present invention is shown in FIG. 2. The medical treatment verification system 210 may reside in, or have portions residing in, any computer such as, but not limited to, the pharmacy computer 104, the central system 108, and/or the digital assistant 118. Therefore, computer 200 of FIG. 2 may be representative of any computer in which the medical treatment verification system 210 resides or partially resides.

Generally, in terms of hardware architecture, as shown in FIG. 2, the computer 200 includes a processor 202, memory 204, and one or more input and/or output (I/O) devices 206 (or peripherals) that are communicatively coupled via a local interface 208. The local interface 208 can be, for example, but not limited to, one or more buses or other wired or wireless connections, as is known in the art. The local interface 208 may have additional elements, which are omitted for simplicity, such as controllers, buffers (caches), drivers, repeaters, and receivers, to enable communications. Further, the local interface may include address, control, and/or data connections to enable appropriate communications among the other computer components.

Processor 202 is a hardware device for executing software, particularly software stored in memory 204. Processor 202 can be any custom made or commercially available processor, a central processing unit (CPU), an auxiliary processor among several processors associated with the computer 200, a semiconductor based microprocessor (in the form of a microchip or chip set), a macroprocessor, or generally any device for executing software instructions. Examples of suitable commercially available microprocessors are as follows: a PA-RISC series microprocessor from Hewlett-Packard Company, an 80x86 or Pentium series microprocessor from Intel Corporation, a PowerPC microprocessor from IBM, a Sparc microprocessor from Sun Microsystems, Inc., or a 68xxx series microprocessor from Motorola Corporation. Processor 202 may also represent a distributed processing architecture such as, but not limited to, SQL, Smalltalk, APL, KLisp, Snobol, Developer 200, MUMPS/Magic.

Memory 204 can include any one or a combination of volatile memory elements (*e.g*., random access memory (RAM, such as DRAM, SRAM, SDRAM, *etc.*)) and nonvolatile memory elements (*e.g*., ROM, hard drive, tape, CDROM, *etc*.). Moreover, memory 204 may incorporate electronic, magnetic, optical, and/or other types of storage media. Memory 204 can have a distributed architecture where various components are situated remote from one another, but are still accessed by processor 202.

The software in memory 204 may include one or more separate programs. The separate programs comprise ordered listings of executable instructions for implementing logical functions. In the example of FIG. 2, the software in memory 204 includes the medical treatment verification system 210 in accordance with the present invention and a suitable operating system (O/S) 212. A non-exhaustive list of examples of suitable commercially available operating systems 212 is as follows: (a) a Windows operating system available from Microsoft Corporation; (b) a Netware operating system available from Novell, Inc.; (c) a Macintosh operating system available from Apple Computer, Inc.; (d) a UNIX operating system, which is available for purchase from many vendors, such as the Hewlett-Packard Company, Sun Microsystems, Inc., and AT&T Corporation; (e) a LINUX operating system, which is freeware that is readily available on the Internet; (f) a run time Vxworks operating system from WindRiver Systems, Inc.; or (g) an appliance-based operating system, such as that implemented in handheld computers or personal digital assistants (PDAs) (*e.g*., PalmOS available from Palm Computing, Inc., and Windows CE available from Microsoft Corporation). Operating system 212 essentially controls the execution of other computer programs, such as the medical treatment verification system 210, and provides scheduling, input-output control, file and data management, memory management, and communication control and related services.

The medical treatment verification system 210 may be a source program, executable program (object code), script, or any other entity comprising a set of instructions to be performed. When a source program, the program needs to be translated via a compiler, assembler, interpreter, or the like, which may or may not be included within the memory 204, so as to operate properly in connection with the O/S 212. Furthermore, the medical treatment verification system 210 can be written as (a) an object oriented programming language, which has classes of data and methods, or (b) a procedural programming language, which has routines, subroutines, and/or functions, for example but not limited to, C, C++, Pascal, Basic, Fortran, Cobol, Perl, Java, and Ada. In one embodiment, the medical treatment verification system 210 is written in C++. In other embodiments, the medical treatment verification system 210 is created using Power Builder. The I/O devices 206 may include input devices, for example but not limited to, a keyboard, mouse, scanner, microphone, touch screens, interfaces for various medical devices, bar code readers, stylus, laser readers, radio-frequency device readers, *etc*. Furthermore, the I/O devices 206 may also include output devices, for example but not limited to, a printer, bar code printers, displays, *etc*. Finally, the I/O devices 206 may further include devices that communicate both inputs and outputs, for instance but not limited to, a modulator/demodulator (modem; for accessing another device, system, or network), a radio frequency (RF) or other transceiver, a telephonic interface, a bridge, a router, *etc*.

If the computer 200 is a PC, workstation, PDA, or the like, the software in the memory 204 may further include a basic input output system (BIOS) (not shown in FIG. 2). The BIOS is a set of essential software routines that initialize and test hardware at startup, start the O/S 212, and support the transfer of data among the hardware devices. The BIOS is stored in ROM so that the BIOS can be executed when computer 200 is activated.

When computer 200 is in operation, processor 202 is configured to execute software stored within memory 204, to communicate data to and from memory 204, and to generally control operations of computer 200 pursuant to the software. The medical treatment verification system 210 and the O/S 212, in whole or in part, but typically the latter, are read by processor 202, perhaps buffered within the processor 202, and then executed.

When the medical treatment verification system 210 is implemented in software, as is shown in FIG. 2, it should be noted that the medical treatment verification system 210 can be stored on any computer readable medium for use by or in connection with any computer related system or method. In the context of this document, a computer readable medium is an electronic, magnetic, optical, or other physical device or means that can contain or store a computer program for use by or in connection with a computer related system or method. The medical treatment verification system 210 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be for example, but not limited to, an electronic, magnetic, optical, electromagnetic, infrared, or semiconductor system, apparatus, device, or propagation medium. More specific examples (a non-exhaustive list) of the computer-readable medium would include the following: an electrical connection (electronic) having one or more wires, a portable computer diskette (magnetic), a random access memory (RAM) (electronic), a read-only memory (ROM) (electronic), an erasable programmable read-only memory (EPROM, EEPROM, or Flash memory) (electronic), an optical fiber (optical), and a portable compact disc read-only memory (CDROM) (optical). Note that the computer-readable medium could even be paper or another suitable medium upon which the program is printed, as the program can be electronically captured, via, for instance, optical scanning of the paper or other medium, then compiled, interpreted or otherwise processed in a suitable manner if necessary, and then stored in a computer memory.

In another embodiment, where the medical treatment verification system 210 is implemented in hardware, the medical treatment verification system 210 can be implemented with any, or a combination of, the following technologies, which are each well known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), *etc.*

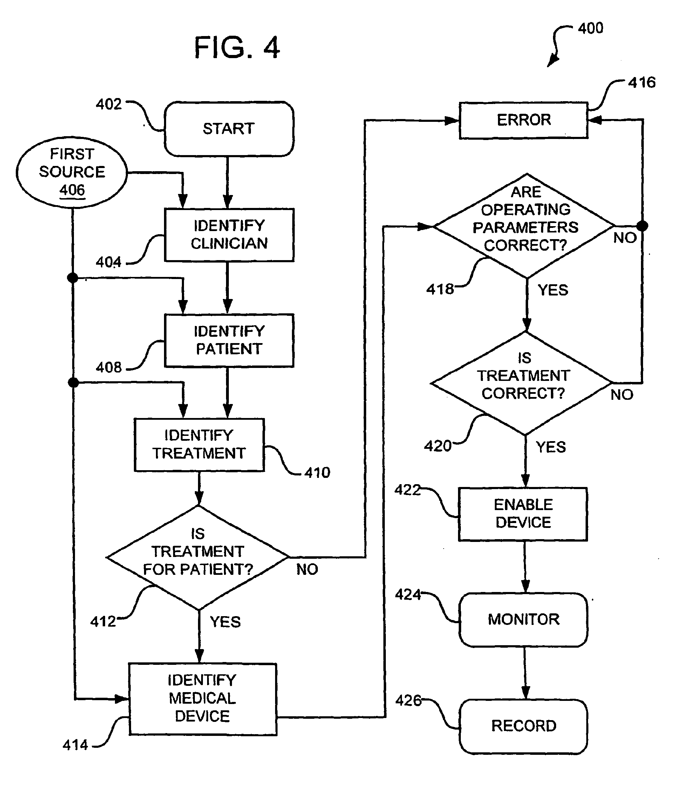

Any process descriptions or blocks in figures, such as FIGS. 3 and 4, should be understood as representing modules, segments, or portions of code which include one or more executable instructions for implementing specific logical functions or steps in the process, and alternate implementations are included within the scope of the embodiments of the present invention in which functions may be executed out of order from that shown or discussed, including substantially concurrently or in reverse order, depending on the functionality involved, as would be understood by those having ordinary skill in the art.

FIG. 3 is a block diagram showing functional components of the patient care system 100 of FIG. 1. FIG. 3 also includes functions blocks for generating infusion orders that include the operating parameters that are verified in the medical treatment verification system 210. Medical treatment verification system 210 may be practiced as a modular system where the modules represent various functions of the medical treatment verification system 210. FIG. 3 presents the *medical treatment* verification system 210 as a *medication* verification system. However, the use of the medical treatment verification system is broader than simply medication verification. The flexibility of the system may be enhanced when the system is practiced as a modular system. The modules of the system may be included in various portions of the patient care system 100. The patient care system 100 includes a medication management module 302, a prescription generation module 304, a prescription activation module 306, and a prescription authorization module 308.

The medication management module 302 may coordinate the functions of the other modules in the patient care system 100 that are involved in the administration of medical treatment. The medication management module 302 will generally coordinate with other portions of the patient care system 100. The medication module 302 may include sub-modules for operating and/or interfacing with a CPOE, for operating and/or communicating with point-of-care modules, and for operating and/or communicating with medical treatment comparison modules. In FIG. 3, an admissions, discharge, and transfer (ADT) interface 310, a billing interface 312, a lab interface 314, and a pharmacy interface 316 are shown. ADT interface 310 may be used to capture information such as the patient's size, weight, and allergies. Pharmacy interface 316 imports orders from the pharmacy. The pharmacy interface 316 may be an HL7 type of interface that interfaces with other systems for entering orders, such as a CPOE. This ability reduces the necessity for entering data into the patient care system 100 more than once. The pharmacy interface 316 may be configured to communicate with commercially available systems such as, but not limited to Cerner, HBOC, Meditech, SMS, and Phamous. Various other interfaces are also known to those having ordinary skill in the art but are not shown in FIG. 3.

The medication management module 302 may have additional features such as the ability to check for adverse reactions due to drug-to-drug incompatibility, duplicate drug administration, drug allergies, drug dosage limitations, drug frequency limitations, drug duration limitations, and drug disease contraindications. Food and alcohol interactions may also be noted. Drug limitations may include limitations such as, but not limited to, limitations associated with adults, children, infants, newborns, premature births, geriatric adults, age groupings, weight groupings, height groupings, and body surface area. Generally, the medication management module 302 will also prevent the entry of the same prescription for the same patient from two different sources within the patient care system 100.

The medication management module 302 may also include the ability to generate reports. The reports include, but are not limited to, end-of-shift, titration information, patient event lists, infusion history, pump performance history, pump location history, and pump maintenance history. The end-of shift report may include the pump channel, start time, end time, primary infusion, piggyback infusion, medication, dose, rate, pump status, volume infused, volume remaining, time remaining, and the last time cleared. The infusion history report includes medications and volume infused.

The medication management module 302 may also include a medical equipment status database. The medical equipment status database includes data indicating the location of a medical device 332 within the patient care system 100. The medical equipment status database may also include data indicating the past performance of a medical device 332. The medical equipment status database may also include data indicating the maintenance schedule and/or history of a medical device 332

The prescription generation module 304 generates hard prescriptions and electronic (E-copy) prescriptions. Hard prescriptions are generally produced in triplicate in medical facilities. A first hard copy 318 is generally sent to the is generally sent to the pharmacy, a second hard copy 320 is generally kept for the patient's records, and third hard copy 322 is sent to treatment location 106. An electronic prescription is sent to the medication management module 302.

A computerized physician order entry (CPOE) system may be employed to carry out some or all of the functions of the functions of the prescription generation module 304. Clinicians 116 may enter data in a variety of manners such as, but not limited to, using a tablet wireless computer, treatment cart 132, and a workstation.

Prescription generation may include calculating the dose based on patient weight and/or height (from the ADT interface 310), the drug amount, diluent volume, concentration, rate. Prescription generation 304 may include confirming operating parameters. The operating parameters may be based on information from a prescription entry module 324. Prescription generation may occur anywhere in the patient care system 100 such as, but not limited to, the pharmacy, the treatment location 106, and a nursing center.

Infusion prescriptions may include prescriptions such as, but not limited to, single dose infusions, intermittent infusions, continuous infusions, sequencing, titrating, and alternating types. Infusion prescriptions may also include total parenteral nutritional admixtures (TPN), chemotherapy continuous infusion, piggybacks, large volume parenterals, and other infusion prescriptions. The patient care system 100 is capable of functioning without end dates for orders. The patient care system 100 may use a continuous schedule generator that looks ahead a predefined time period and generates a schedule for admixture filling for the time period. The predefined time period may be defined at the patient care system 100 level or at subsystem levels such as the clinical discipline level and an organizational level. The predefined time periods may be adjustable by the clinician 116 entering the order. The schedule may be automatically extendable as long as the order is active in the patient care system 100.

The medication management module 302 may interface with more than one prescription generation module 304. The medication management module may receive orders from anywhere within the patient care system 100.

The pharmacy computer 104 is able to access the electronic copy from the medication management module 302. The prescription activation module 306 is a computer assisted system for coordinating the filling and labeling of prescriptions. The filling of the prescription and the creation or location of medication 124 from stock is handled by the prescription activation module 306.

While activating the prescription, the prescription activation module 306 may calculate the flow rate, if not specified in the prescription, the number of solutions/bags required for a specified period of time, the time period over which each solution/bag is to be administered, and the total volume of each solution/bag based on the concentration of the ingredients in the solution. Flow rates, volume to be infused, and/or duration may be adjusted in the system 100 will automatically calculate dependent quantities, based on calculations, if the maximum dosage for the ingredients in the concentration would be exceeded as identified in the ingredient's medication file, the patient care system 100 will alert the pharmacist and/or clinician 116 and may ask for a reason code for the adjustment.

The patient care system 100 may bypass the prescription activation module 306. This may occur if the clinician, such as the patients' physician, has the authority to immediately activate an order. If the order is immediately activated, the medication management module may go directly to prescription labeling module 326.

In block 326, the patient care system 100 prints the medication label 124. The prescription may be printed remotely and will often be printed by the pharmacy printer 104d. After block 326, the patient care system goes to block 328. In block 328, the medication label 124a is attached to the medication 124. The pharmacist generally provides a visual verification 334 that the medication label 124a matches the first hard copy 318 of the prescription. FIG. 3 shows that a visual verification 334 is also associated with prescription authorization module 308. The medication 124 may then be transported from the pharmacy to the treatment location 106. A portable medical treatment cart 132 may be used for a portion of the route from the pharmacy to the treatment location 106.

The medication label 124a may include information for admixing. If not generated within patient care system 100, medication label 124a may be provided by a bulk medication supplier. If provided by a bulk medication supplier, the patient care system 100 has the capability of coordinating gathering the information from the medication label 124a. In addition, the patient care system 100 has the ability to add information, such as a patient identifier, to medication label 124a.

The medication labeling module 328 places the medication label 124 on the medication 124. This may be accomplished manually. This may also be accomplished using an automatic prescription filling and packaging system (not shown). If an automatic filling and packaging system is used, medication labeling module 328 provides data for coordination of the labeling of the medication 124 to the filling and packaging system.

At the treatment location 106, the clinician 116 uses a wireless device 330, such as digital assistant 118 and/or medical treatment cart 132, to verify and administer medication 124 to the patient 112. Wireless device 330 communicates with the medication management module 302 via a communication path, such as first communication path 126.

Clinician 116 generally identifies his/herself by scanning her badge 116a, identifies the patient 112 by scanning wristband 112a, identifies the medication 124 by scanning medication label 124a, and identifies the medical device 332, such as infusion pump 120, by scanning label 120d. The clinician 116 may also identify him/herself by providing a fingerprint and/or password. The medical device 332 may be a medical device capable of two-way communication with the medication management module 302. Alternatively, the medical device 332 may only be capable of providing information to the medication management module 302. The medical treatment verification system 210 assists the clinician 116 in administering and verifying the medical treatment. The medical treatment verification system 210 will generally result in the downloading of operating parameters to the medical device 332. The clinician 116 may generally provide a visual verification to confirm the third copy 322 and/or the MAR matches the labeled medication 124. Scanner 338 may be used to enter machine readable information from the third copy 322 to the wireless device 330 and the medical device 332.

The patient care system 100 includes the ability to make adjustments and modifications to infusion orders. Among other modules that may include the ability to make infusion adjustments are prescription entry 324, prescription activation 306, prescription authorization 308, and prescription modification module 336. Clinician 116 may access prescription modification module 336 in order to make adjustments to an order. The clinician 116 may access the prescription modification module 336 throughout the patient care system 100. However, one very useful location for the clinician 116 to access the prescription modification module 336 is in the treatment location 106.

In the prescription authorization module 308, the patient care system 100 determines whether the clinician 116 has the authority to independently modify an infusion order. The clinician 116 may be recognized by the patient care system 100 as having the authority to independently modify certain portions of the order. If the clinician 116 does not have the authority to independently modify the order, a pharmacist or physician may be requested to approve the modification entered by the clinician 116.

FIG. 4 is a flowchart showing a first exemplar embodiment 400 of the medical treatment verification system 210 of FIG. 2. The medical treatment verification system 400 is called in block 402. In block 404, the system 400 accesses information related to the identity of the clinician 116. A first source 406, such as digital assistant 118 may provide information related to the identity of the clinician 116. Digital assistant 118 may acquire the information by reading the clinician's badge 116a with a bar code reader. First source 406 may also be another computer located at the remote location. First source 406 may be other sources of information such as, but not limited to, a bar code, such as a bar code included in clinician's badge 116a, a tag, laser readable data, radio-frequency readable data, a keyboard, an iB utton reader, a fingerprint scanner, and a bar code reader that is not associated with digital assistant 118. Block 404 may include converting a signal generated by first source 406 to a computer readable medium format. Block 404 may also include using the information provided by first source 406 to match the information to the identity of the clinician 116 through the use of a look-up table stored in memory 204. After block 404, the system 400 goes to block 408.

In block 408, the system 400 identifies the patient 112. First source 406 may provide information related to the identity of the patient 112. Digital assistant 118 may acquire the information by reading the patient's wristband 112a with a bar code reader. Block 408 may include converting a signal generated by first source 406 to a computer readable medium format. Block 408 may also include using the information provided by first source 406 to match the information to the identity of the patient 112 through the use of a look-up table stored in memory 204 or any other matching process. After block 408, the system 400 goes to block 410.

In block 410, the system 400 identifies the treatment. The treatment may be the administration of medication 124. First source 406 may provide information related to the identity of the treatment. The identity of the treatment may be the identity of a medication 124. The medication identity may be correlated with a medication identifier. The medication identifier may include information such as, but not limited to, a medication identification number, a mixture identification number, a patient 112 encounter number, a drug name, a dosage, a manufacturer, a batch, an expiration date, and/or a drug prescriber. In block 410, any edubytes, messages, hazard warnings, and/or administrative instructions may be displayed on the digital assistant 118. Administrative instructions may include specialty set, filter requirements, warnings, and precautions. In block 410, if the medical treatment is a medication, the system 400 may check for expirations, such as the expiration of an admixture and lot recalls.

Digital assistant 118 may acquire the information by reading medication label 124a with a bar code reader. Block 410 may include converting a signal generated by first source 406 to a computer readable medium format. Block 408 may also include using the information provided by first source 406 to match the information to the identity of the medical treatment through the use of a look-up table stored in memory 204 or other matching algorithms. After block 410, the system 400 goes to block 412.

In block 412, the system 400 determines whether the medical treatment has been previously associated with patient 112. The determination will often be made by the device that gathers data related to the identity of the patient and the medical treatment. For example, a clinician 116 may use the digital assistant 118 as the first source 406 to read a bar code from a patient's wristband 112a. The clinician 116 may then use the digital assistant 118 to read medication label 124a. The digital assistant 118 may then determine whether the patient identifier from the patient's wristband 112a is equivalent to the patient identifier from the medication label 124a.

One manner of previously associating the medical treatment with the patient is to associate the patient and the medical treatment in the central system 108 and/or in the pharmacy system 104. A physician may make the association through a computerized prescription ordering system. A pharmacist may make the association by entering a patient identifier and a medication identifier in the pharmacy system 104 where the medication identifier includes the patient identifier. The patient identifier may be derived from input sources such as, but not limited to, admission records, orders, an electronic physician order entry system, and/or prescriptions.

If the system 400 determines the medical treatment has not been previously associated with patient 112, the system 400 moves to block 416 where an alarm/error status is provided by the system 400. Block 416 may include displaying the alarm/error status on the digital assistant 118. If the system 400 determines the medical treatment has been previously associated with patient 112, the system 400 moves to block 414

In block 414, the system 400 identifies the medical device. The medical device is configured to be the type that delivers the medical treatment to the patient. For example, the medical device may be infusion pump 120 if the medical treatment is medication 124. First source 406 may provide information related to the identity of the medical device. Digital assistant 118 may acquire the information by reading label 120d with a bar code reader. Block 414 may include converting a signal generated by first source 406 to a computer readable medium format. Block 414 may also include using the information provided by first source 406 to match the information to the identity of the medical device through the use of a look-up table stored in memory 204 or other matching algorithm.

Block 414 may include identifying sub-systems of the medical device. For example, if the medical device is an infusion pump, the infusion pump may have multiple channels. The channels may have barcodes. The channels may be associated with a primary medication and a "piggyback" medication. Block 414 may include identifying these sub-systems, including piggybacks. After block 414, the system 400 goes to block 418.

In block 416, the medical verification system 400 provides an alarm/error status signal. The alarm/status signal may be triggered by a variety of circumstances such as, but not limited to, the system 400 does not recognize the patient, the system 400 does not recognize the treatment, the system 400 cannot match the treatment to an order, the system 400 cannot identify the medical device 332, the operating parameters are not equivalent, and the treatment parameters are outside the treatment tolerances. The treatment tolerances may be define at the patient care system 100 level or as a subset of the patient care system 100.

In block 418, the medical treatment verification system 400 determines whether the operating parameters are correct. The operating parameters are correct if they are consistent with a verified medical treatment. The system 400 may include the downloading of operating parameters to the medical device. The operating parameters may be downloaded from a variety of sources such as, but not limited to, pharmacy computer 104, medication label 124a, digital assistant 118, and the clinician 116 may manually enter the operating parameters. One check that may be performed is to confirm the dose is greater than preset tolerances. The operating parameters may be parameters such as, but not limited to, a flow rate per unit of time, a quantity of medication, a dosing unit, a dosing duration, a dosing volume, a drug name, a dose unit, and a monitoring limit. The dosing information may be provided directly or based on patient 112 attributes such as patient weight.

If the operating parameters are not correct, the medical treatment verification system 201 goes to block 416 and returns an error message. If the operating parameters are correct, the medical treatment verification system 400 may display the flow rate and dose information. The display may appear on display 120c, and/or digital assistant 118.

In block 420, the medical treatment verification system 400 determines whether the treatment is correct. The treatment is correct if the treatment is for the patient, the treatment includes the right medication 124, the treatment includes the correct amount of medication, and the treatment is being administered at the right time. The clinician may also be queried to verify the right route by visually inspecting the medical device and related equipment. The clinician 116 may change some parameters, such as the timing of the medical treatment. If changed, a record of the change is generally kept in the patient care system 100. If the medical treatment verification system 400 determines the treatment is not correct, the system 400 goes to block 416 and provides an error message. If the medical treatment verification system 400 determines the treatment is correct, the system 400 goes to block 422.

Among the factors that may be considered in determining whether the treatment is correct, the system 400 may look to general rules, "look-alike" checks, and "sound-alike" checks. The general rules may include rules, such as but not limited to, a maximum flow rate that applies to all medications throughout the treatment facility, a maximum flow rate for the medication, and general rules based on patient characteristics, and a combination of these rules.

In block 422, the medical treatment verification system 400 enables the medical device. This may include the clinician 116 providing a start signal to begin the infusion. In the event the medical device is in delayed start mode, block 420 may include providing a signal that the operating parameters have been downloaded and the medical device should provide the treatment as soon as the delay period is over.

In block 424, the medical treatment verification system 400 monitors the administration of the medical treatment. While the system 400 monitors the medical treatment administration, any changes to the operating parameters of the pump may be reflected throughout the patient care system 100 within 10 seconds. The changes may be noted on the digital assistant 118. During the infusion, the clinician 116 can adjust the infusion parameters. The adjustment may be to the flow rate of the infusion. Clinician 116 generally has the authority to adjust the flow rate within predefined boundaries. This allows the clinician 116 to "catch-up" if the infusion tubing is blocked or other interruptions occur.

In block 426, the medical treatment verification system 400 records the result of the medical treatment administration. The result may be the successful administration of the medical treatment pursuant to the operating parameters. However, other results are possible such as, but not limited to, a patient's refusal to accept the medical treatment, a modification of the medical treatment, and equipment malfunction, and an interstitial infusion error. In the case of a modification of the medical treatment, a modified order may be generated. The modified order may then be linked in the medication management module 302 with the original order.

Various blocks of the medical treatment verification system, such as blocks 418 to 424, may include displaying treatment information on the digital assistant 118. This may include displaying information that mirrors the information on display 120c of infusion pump 120. The information on display 120c of infusion pump 120 may be supplemented with information about the patient, the patient location, and the infusion order. This information may include information regarding multiple channels of infusion pump 120. The displayed information may include information such as, but not limited to, personality, prompt line, status line, operating icons and pump head display. Operating icons include falling drop, stop sign, flow check piggyback, Guardian, and delay start. The pump head display includes information such as the drug label and the infusion rate. Those having ordinary skill in the art are familiar with the displayed information and operating icons described above.

In another embodiment, the medical treatment verification system 210 may determine there is no information stored in the patient care system related to the medical treatment the clinician 116 desires to administer to the patient 112. If the patient care system 100 recognizes the clinician 116 as having the authority to initiate the desired medical treatment, the system may allow for the administration of the medical treatment without going to block 416.

Throughout this document and the related claims, "central location" and "remote location" are relative terms to each other. A "remote location" is any location where a patient is receiving treatment through a controlled medical device, such as a patient treatment location 106 where patient 112 is receiving treatment through an infusion pump 120. "Central location" is any location, other than the remote location, where parameters for operating the medical device are accessible such as, but not limited to, the location of the pharmacy computer 104 and the central system 108. In a typical arrangement, several remote locations, such as treatment location 106, are in communication with a central location.

Downloading of operating parameters may include determining whether the patient identifier associated with the medical treatment and/or the patient identifier retrieved from the wristband 112a, is the same as the patient identifier associated with the medical treatment at the central location. The determination will often be made by the first computer, for example, the pharmacy computer 104a. If the system 400 determines the various patient identifiers are not the same the system may move to block 416. If the system 300 determines the various patient identifiers are the same, the system 400 may download the operating parameters directly to the medical device. The system 400 may send the operating parameters to a medical device such as infusion pump 120.

One benefit of the medical treatment verification system 210 is that the operating parameters for the medical device do not have to pass through digital assistant 118, or any other computer in the remote location prior to the operating parameters being available to program the medical device. Bypassing computers at the remote location eliminates a potential source of errors in administering medication 124 to a patient 112. The operating parameters for the medical device may be sent "directly" to the medical device assuming the various verifications are achieved. In this context, "directly" meaning that the operating parameters may be sent to the medical device without passing through the digital assistant, or any other computer in the remote location.

In another embodiment, the system 400 may include an additional block (not shown) where the central computer accepts a second medication identifier. The second medication identifier may be entered by the clinician 116 at the remote location. The second medication identifier may be a revised first medication identifier. For example, the second medication identifier may be part of the prescription or electronic physician order entry that is the source for the first patient ID and the operating parameters The system 300 may then confirm the first and second medication IDs are equivalent prior to sending the operating parameters to the medical device. The second medication ID may be replaced by a revised first medication ID between the time the prescription is entered and the time the medication 124 arrives at the treatment location 106. The system 400 will then sound an alarm if second medication identifier is not equivalent to the first medication identifier that was included in the medication label 124a.

In a former embodiment, the system 400 may include an additional block (not shown) where the operating parameter is used to program the medical device.

In one implementation of system 400, an order is entered in pharmacy computer 104. The order includes a first patient identifier and an operating parameter. The pharmacy computer 104 generates a medication label 124a. that is affixed to medication 124. The medication 124 is sent to a treatment, location 106. At treatment location 106, clinician 116 reads the clinician's badge 116a, patient's wristband 112a, and medication label 124a with a digital assistant 118. The digital assistant 118 determines whether medication label 124a and wristband 112a identity the same patient 112. The system 400 then sends the medication identifier to the pharmacy computer 104. The pharmacy computer 104 confirm the medication label 124a identifies the same patient as the order and sends the operating parameter to an infusion pump. The operating parameters may be sent directly to the infusion pump. The operating parameter is then used to program the infusion pump to administer the medication 124 to the patient 112.

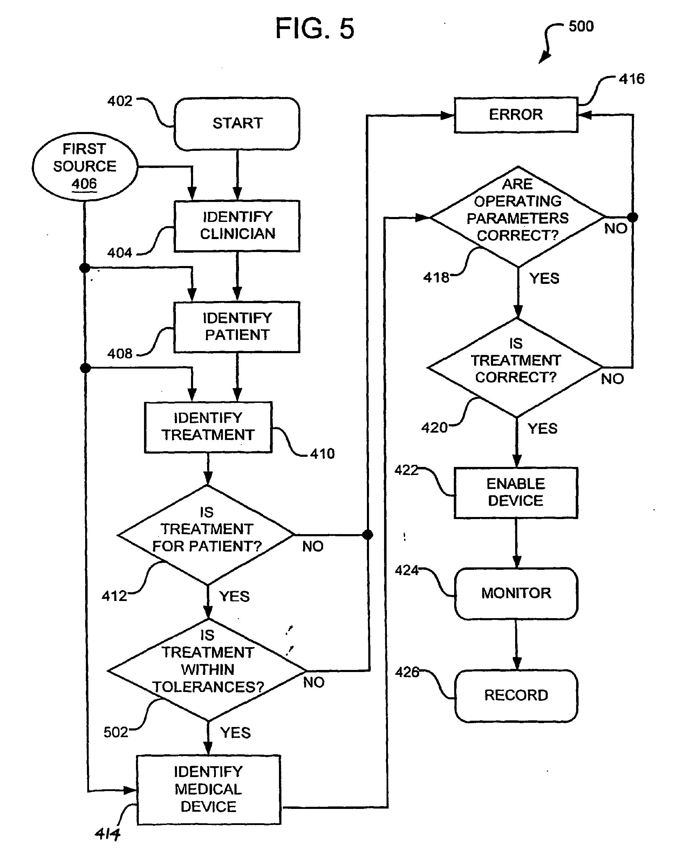

FIG. 5 is a flowchart showing a second exemplar embodiment 500 of the medical treatment verification system 210 of FIG. 2 .The medical treatment verification system 500 is called in block 402. The system 500 then proceeds through functions similar to blocks 404 through 412 as described in regard to embodiment 400. From block 412, the medical treatment verification system 500 moves to block 502.

In block 502, the medical treatment verification system 500 determines if the treatment is within tolerances. The system 500 may include variety of tolerances such as, but not limited to, infusion flow rate tolerances. If the system 500 determines the treatment is not within tolerances, the system moves to block 416 and provides an error signal. The system 500, may also offer the ability to modify the treatment and/or the tolerances. If the system 500 determines the treatment is within tolerances, the system goes to block 414 and continues as described in regard to FIG. 4.

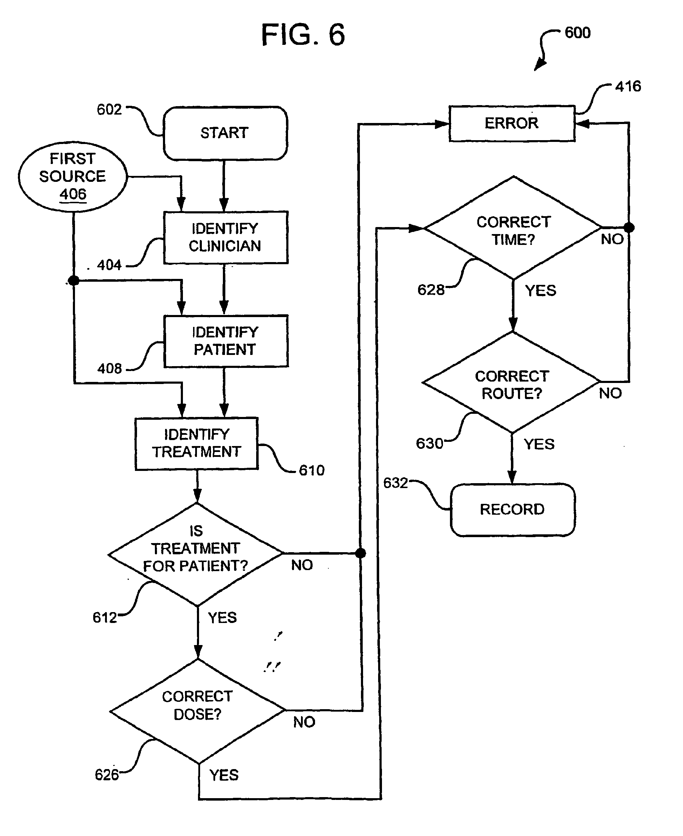

FIG. 6 is a flowchart showing a comparative arrangement 600 of the medical treatment verification system 210 of FIG. 2. Comparative arrangement 600 may be used with medical treatment that do not include infusion order such as, but not limited to, oral medications. The medical treatment verification system 600 is called in block 602. The system 600 then proceeds through functions similar to blocks 404 through 408 as described in regard to embodiment 400. From block 408, the medical treatment verification system 600 moves to block 610.

In block 610, the system 600 identifies the treatment. The treatment may be the administration of an oral medication. First source 406 may provide information related to the identity of the treatment. The identity of the treatment may be the identity of the oral medication. The identity may be coded onto the medication and/or onto packaging for the medication. The oral medication identity may be correlated with a medication identifier. In block 610, if the medical treatment is a medication, the system 600 may check for expirations and recalls.

Digital assistant 118 may acquire the information by reading the oral medication label with a bar code reader. Block 610 may include converting a signal generated by first source 406 to a computer readable medium format. After block 610, the system 600 goes to block 612.

In block 612, the system 600 determines whether the medical treatment has been previously associated with patient 112. The determination will often be made by the device that gathers data related to the identity of the patient and the medical treatment. For example, a clinician 116 may use the digital assistant 118 as the first source 406 to read a bar code from a patient's wristband, 112a. The clinician 116 may then use the digital assistant 118 to read the oral medication label. The digital assistant 118 may then determine whether the patient identifier from the patients wristband 112a is equivalent to the patient identifier from the medication label.

If the system 600 determines the medical treatment has not been previously associated with patient 112, the system 600 moves to block 416 where an alarm/error status is provided by the system 400. Block 416 may include displaying the alarm/error status on the digital assistant 118. If the system. 600 determines the medical treatment has been previously associated with patient 112, the system 600 moves to block 626.

In block 626, the system 600 determines if the treatment is the correct dose. The dose is correct if they are consistent with a verified medical treatment. The system 400 may include downloading instructions for the delivery of the medical treatment to the digital assistant 118 and/or the medical treatment cart 132. The instructions may be downloaded from a variety of sources such as, but not limited to, pharmacy computer 104, the oral medication label, digital assistant 118 (to the cart 132), medication cart 132 (from digital assistant 118), and the clinician 116 may manually enter the dose from the medication label. One check that may be performed is to determine if the dose is greater than preset tolerances. The instructions include instructions such as, but not limited to, a quantity of medication, a medication name, and a dose unit. The dosing information may be provided directly or based on patient 112 attributes such as patient weight

If the dose is not correct, the medical treatment verification system 600 goes to block 416 and returms an error message. If the dose is correct, the medical treatment verification system 600 may display the dose information. The display may appear on display 120c, digital assistant 118, and/or cart 132.

In block 628, the system 600 determines if the treatment is being administered at the correct time. If the treatment is not being administered at the correct time, the system goes to block 416 and returns an error message. If the medical treatment is being administered at the correct time, the system 600 goes to block 630.

In block 630, the system 600 determines if the treatment is being administered by the correct route. If the treatment is not being administered by the correct route, the system goes to block 416 and ... returns an error message. If the medical treatment is being administered at the correct time, the system 600 goes to block 632.

In block 632, the medical treatment verification system 600 records the result of the medical treatment administration. The result may be the successful administration of the medical treatment, pursuant to the instructions. However, other results are possible such as, but not limited to, a patient's refusal to accept the medical treatment, a modification of the medical treatment, and equipment malfunction. In the case of a modification of the medical treatment, a modified order may be generated. The modified order may then be linked in the medication management module 302 with the original order.

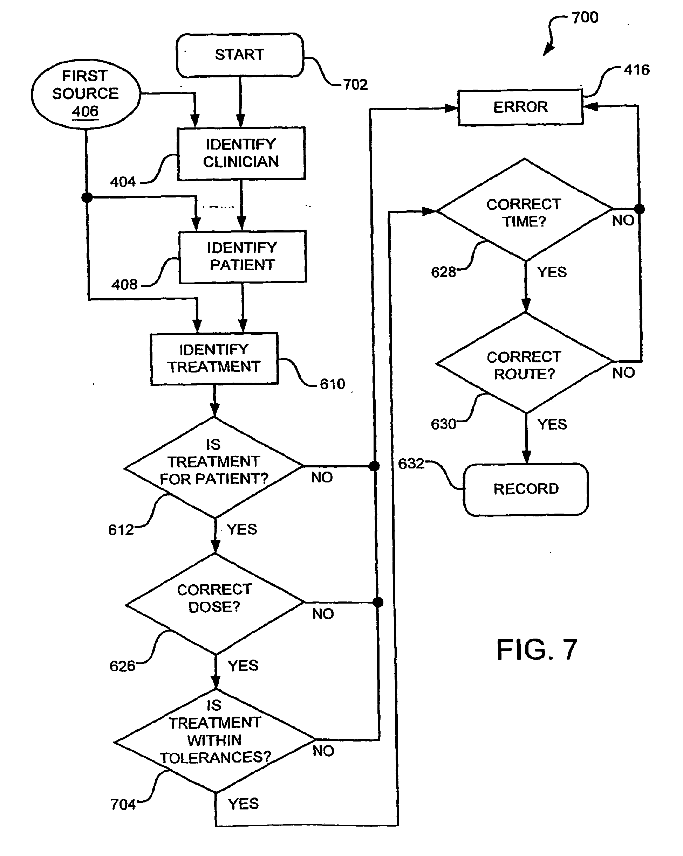

FIG. 7 is a flowchart showing a another comparative arrangement 700 of the medical treatment verification system 210 of FIG. 2. The medical treatment verification system 700 is called in block 702. The system 700 then proceeds through functions similar to blocks 404 through 408 as described in regard to embodiment 400. The system 700 then proceeds through functions similar to blocks 612 through 626 as described in regard to comparative arrangement 600. From block 626, the medical treatment, verification system 700 moves to block 712.

In block 712, the medical treatment verification system 700 determines if the treatment is within tolerances. The system 700 may include a variety of tolerances such as, but not limited to, dosage tolerances. If the system 700 determines the treatment is not within tolerances, the system moves to block 416 and provides an error signal. The system 700, may also offer the ability to modify the treatment and/or the tolerances. If the system 700 determines the treatment is within tolerances, the system goes to block 628 and continues as described in regard to FIG. 6.

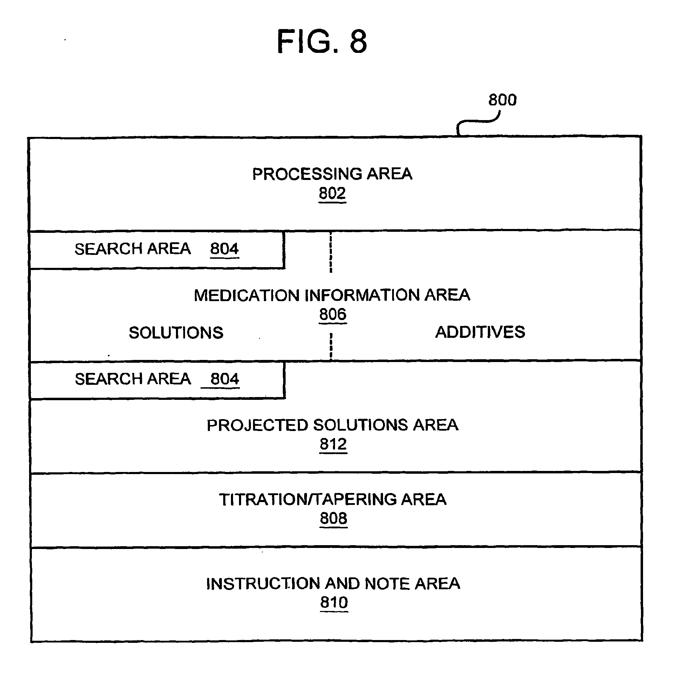

FIG. 8 is an exemplar computer screen 800 that is useful in implementing various functions of the patient care system 100. In addition to other functions, computer screen 800 may be used to enter new infusion medication orders, to modify existing medication orders, and to cancel medication orders. Computer screen 800 includes a processing area 802, search areas 804, a medication information area 806, a titration/Tapering criteria area 808, an instruction and note area 810, and a projected solution ingredient area 812. Infusion medication order types include single dose, intermittent, continuous, sequencing, and alternating. Computer screen 800 may be used with digital assistant 118, pharmacy computer 104, infusion pump 120, a CPOE system, and medical treatment cart 132. Compute screen 800 will generally be designed to have the look-and-feel of clinician 116 accessible computer screens throughout the patient care system 100. The functions of computer screen 800 are partially accomplished with database linkage techniques that are familiar to those having ordinary skill in the art such as, but not limited to, hyperlinks, definition boxes, and dropdown menus.

The processing area 802 may include the ability to trigger a new infusion order, a save of the infusion order, and a cancellation of an infusion order. A clinician 116 may customize computer screen 800 to provide clinician 116 favorite order entry procedures. The processing area 802 includes a status indicator for orders. The processing area 802 includes an area for indicating whether a PRN order (a "when necessary" order) may be placed by clinician 116. The processing area 802 also includes the ability to display and adjust operating parameters, infusion order route, infusion line, infusion administration site, infusion order start time, infusion medication order type, infusion flow rate tolerance, infusion flow rate, infusion duration, area of preparation (such as pharmacy or a remote site). The processing area 802 may also include an area for linking medical orders to other medical orders such as, linking a physician's infusion order to another medical order that may be entered by another clinician 116. The processing area 802 may include a trigger for displaying data in other areas of the computer screen 800 such as, but not limited to the projected solutions area 812.

Search areas 804 allow for searching for medications, solutions and/or additives for infusion orders. Default diluents may be provided for orders. If a default dosage for a medication is defined in the patient care system 100, the default dosage will automatically appear with the search result that includes the medication. A search from search area 804, will generally produced the medication name, the route of administration, the cost, the package size, the dosage form, the generic name, whether the medication is a narcotic, whether the medication is controlled, whether formulary, and whether the medication is manufactured.

Medication information area 806 is used to define infusion order additives and solutions. Medication information area 806 may include separate additive areas and solution areas. The solution area may include a label "Solution/Diluent". The patient care system 100 may use a medication 124 database, a solutions database, and an additive database to populate the medication information area 806 with medications124, solutions, and additives. Substances identified in one database may also be identified in other databases. The databases may be linked to provide default values for combinations of the medications 124 and solutions.

Titration/Tapering criteria area 808 generally applies to continuous infusion orders. Titration defines certain parameters of an order such as dosage and/or flow rate. Dose and flow rate can be entered as an absolute. Also, mathematical symbols such as, but not limited to, greater than ">", less than "<", and equal "=", may be used alone or in combination to enter information in titration/tapering criteria area 808. A calendar may also be used to enter data in titration/tapering criteria area 808. Dosage and flow rate can also be entered as an acceptable range. Titration/Tapering criteria area 808 may be hidden when non-continuous infusion orders are entered and/or modified.

Instruction and note area 810 includes the ability to save information such as physician notes regarding a patient 112 and/or an infusion order. The instruction and note area 810 may include a display and lookup area for identifying clinicians 116 that are responsible for the patient 112, such as the patient's physician.

The projected solutions area 812 displays solution schedules and related ingredients based on the current state of the order being processed for patient 112. The time period projected may be a patient care system 100 default. The time period may also be adjustable by the clinician 116. The projected solutions area 812 may include an adjustable display indicating the time period projected by the patient care system 100. The data displayed in the projected solutions area will generally be saved when an order save is triggered in the processing area 802. The projected solutions area 812 may include the ability to look back over a period of time while modifying a previously entered order. This allows the clinician 116 to view solutions that may have already been prepared according to the unmodified infusion order.

The patient care system 100 includes the ability to make adjustments and modifications to infusion orders. These adjustment and modification functions are included in prescription modification module 336 (FIG. 3). However, adjustment and modification functions are also accessible from other portions of the patient care system 100 such as, but not limited to prescription entry 324, prescription activation 306, and prescription authorization 308.

The patient care system 100 may include adjustable predefined flow rate adjustment tolerances. The flow rate adjustment tolerances are optionally defined for all organizational levels of the patient care system 100. The tolerances may be for the entire patient care system 100, or for subsystems of the patient care system 100. For example, different flow rate adjustment tolerances may apply to subsystems such as, but not limited to, neonatal, pediatric, psychiatric, specific nursing units, and for specific patients. The flow rate adjustment tolerances can be specified relative to the original ordered flow rate or relative to the immediately preceding flow rate. The clinician 116 may also specify a flow rate tolerance specific to a particular order. The system 100 may include a pre-defined indication of whether the nurse is permitted to override the flow rate adjustment tolerance without requiring a new order. This indication can apply to the entire patient care system 100, a subsystem, or an individual clinician 116.

Medications may have flow rate tolerances. The system 100 may include flow rate override reason codes. Flow rate override reason codes are notations that the clinician 116 may choose from and/or supply if the clinician 116 needs to change the flow rate beyond the bounds defined by the flow rate tolerance. The system 100 may include a defined setting indicating whether or not a message should be sent to the patient's physician if a clinician 116 overrides a flow rate tolerance defined in the order. The system 100 may also include a defined setting indicating whether or not a message should be sent, and to whom, if a clinician 116 overrides the tolerances, such as flow rate tolerances, defined at a level other than the order.

The system 100 may include translation functions such as, but not limited to, flow rate translation, varying ingredient translation, and varying flow rate translation. Flow rate translation includes translating an infusion order into a flow rate defined by volume/time where the order is originally specified in any way such as, but not limited to, dosage/time with a particular concentration, volume per unit of weight/time, dosage per unit of body surface area/time, and total dosage and duration.

Varying ingredient translation includes translating of orders with varying ingredients into the flow rate for the current solution being infused. Orders with varying ingredients include orders such as, but not limited to, sequencing orders. In sequencing orders, different bags have different ingredients and potentially different flow rates.

Varying flow rate translation includes translation of orders with varying flow rates into the flow rate for the current solution being infused. Varying flow rate orders include orders such as, but not limited to, tapering dose orders and alternating dose orders.

The system 100 may include predefined infusion flow rates. The predefined infusion flow rates may have descriptions to permit selection from a drop-down list as a shortcut from keying in the flow rate. The system 100 may include automatic translation between duration and volume to be infused.

The system 100 may also include settable precision displays. Settable precision displays may include displays such as, but not limited to, flow rate displays, volume displays, administration period displays. Flow rate displays may be set to display the flow rate to a set number of decimal places. Various parts of the patient care system 100 may independently determine the precision for flow rates displayed. For example, the system 100 may display to one decimal place for an adult treatment location, and to three decimal places for a neonatal treatment location. Volume displays may similarly be set to display infusion volumes to a set number of decimal places. Settable administration period displays may be used to calculate the administration period based on flow rate if the infusion is a single dose infusion or an intermittent infusion.

The system 100 may also include defined settings such as, but not limited to, a maximum infusion duration setting, an order entry maximum infusion duration override availability setting, and an administration maximum infusion duration override availability setting. The maximum infusion duration setting may be separately definable for the various portions of the patient care system 100. The maximum infusion duration setting may also be specific to a particular medication 124. A maximum infusion duration override availability setting may be provided to set whether it is permissible to override the maximum infusion duration setting at the time of order entry. An administration maximum infusion duration override availability setting may be provided to set whether it is permissible to override the maximum infusion duration at the time of administration and which group of users is allowed to do so. If it is permissible to override during order entry and/or administration, the system 100 may define a subset of the clinicians 116 that have the authority to override the maximum infusion duration setting.

The system 100 also calculates the time remaining for an order to be completed and the volume of an infusion order that remains to be administered. When the clinician 116 uses the system 100 to administer the infusion order, to make flow rate changes, and to check on the status of an infusion, the system 100 calculates time and volume remaining to be administered and indicates if the calculation indicates a partial bag will be used. For example, on the last bag of an order that is to be stopped before the full volume is administered, and/or on a bag within an order that must be changed before the full volume is administered, the clinician 116 is alerted on digital assistant 118 and/or cart 132. The alert may include a message such as "Please only administer 150 ml."

For titrate PRN orders, the clinician 116 is automatically notified of required flow rate changes if the titration conditions in the order indicate that the flow rate must be changed. The system 100 includes defined values for calculating a conversion of flow rates into drip rates. The system 100 defined values may be adjustable. The system 100 may include automatic translation of flow rate to drip rate to assist the clinician 116 during administration of the treatment.

The system 100 includes the ability to document a change in the IV infusion rate. The system may be configured to assist the clinician in capturing all changes in flow rate as the changes are occurring. The clinician 116 may change the flow rate as called for in the order, such as to decrease a morphine infusion rate from 4ml to 2 ml. Though the system 100 may recognize the change as a new order, the system 100 may be configured to avoid duplication so that the modified order does not result in the generation of a new bag.

Clinicians 116 may also change an infusion rate without an order if the patient 112 is complaining of discomfort or to facilitate fluid balance, such as when a the patient 112 is vomiting.

The system 100 has the ability to document changes such as, but not limited to, the ability to document that an infusion has been stopped temporarily, discontinued, and/or restarted. The clinician 116 may stop infusion for a variety of reasons, such as the infusion site having been compromised, the infusion has been dislodged, and/or the infusion may be heparin/saline locked to facilitate the movement of patient 112. The infusion may be resumed when a new site/infusion has been reestablished. However the length of time this may take is variable and is generally recorded by the system 100.

The system 100 includes the ability to document multiple infusions for multiple infusion sites in full detail. In many situations a patient 112 may have multiple medications 124 and "y-ed" infusions so that the some infusions are running into one site and other infusions are infusing into another site. For example, morphine infusion, antibiotics and normal saline infused into the right arm (site 1) and TPN and 2/3 & 1/3 running into a double lumen CVL (site 2). The system 100 allows clinician 116 to have to document which site the various fluids are infusing through. In ICU's, many more than two infusions may be running into one line or one lumen. Clinicians 116 are able to indicate which lumen of a CVL the infusion or medication is running into.

The system 100 includes the ability to document the site location for infusions and any site location changes. Infusion sites are frequently changed due to occlusions or policy. Therefore, clinicians 116 must document a change in the site location if an infusion becomes dislodged and was subsequently restarted.

A method of administering an infusion is described below. The method includes the ability to modify the infusion order. The modifications include modifications to the flow rate, the infusion site, temporary stops to the infusion, restarting the infusion, and hanging a new medication 124 container. The method includes: Scanning a bar code associated with the patient; Scanning a bar code associated with the medication; If the infusion is an admixture, validating the expiry; Selecting a reason for the modification; and recording the remaining volume of the infusion bag or accepting the value calculated from the previous volume and flow rate. The validation of the expiry of the admixture may include the use of an admixture table and/or a barcode.

The reason for the modification may come from a defined table. The reason for the modification may also include a hard-coded value for physician-ordered changes. When the hard-coded value is selected, the clinician 116 is prompted to select the physician from a list of physicians. The attending physician may be the default in the list of physicians.

There may be a quick select feature to halt the administration of the medication 124. If the quick select is not chosen, the following steps may be included. Recording the flow rate and/or accepting the previous value for the flow rate. The previous value is generally displayed on the digital assistant display 118a, the infusion pump display 120c, and/or the medical cart 132. Comparing the previous flow rate to the ordered flow rate. This comparison may be accomplished by using system 100 or subsystem rules and tolerances. Displaying appropriate messages. Conversions between flow rates and drip rates may be displayed. The conversions may be calculated based on system 100 defined drip-rate conversion tables. The system 100 typically uses descriptions based on the tubing used to make it easy for the clinician 116 to select the correct drip rate conversion. Changing the flow rate trigger the system to automatically: Validate the expiry of this solution based on scheduled flow rate; If the solution expires before or during the administration, give a message to the clinician, such as " This solution will expire during the scheduled administration period. Please contact the pharmacy"; if a premixed solution and/or a customized solution, validate the expiry by parsing the scan code, if possible; Accept the previous infusion site or select a new infusion site location from a list or a graphical anatomical representation; and, recalculating the schedule to implement pharmacy restocking.

The system 100 may also include a system 100 or subsystem defined table defining override codes for administering infusion orders at a time other than the time specified in the original order. If the clinician 116 administers a drug outside the defined administration time tolerance, the clinician 116 may be required to choose a reason code for the modification.

The system 100 may also include a process to request a PRN Infusion to be prepared and delivered. This option may include prompting the clinician 116 to select a PRN infusion from a list of PRN orders placed for the patient; and defaulting the requested infusion bags to one, but the clinician 116 has authority to modify the requested quantity.

The system accesses information related to the identity of the clinician 116, as was previously described in reference to block 404 (FIG. 4). The system 100 may identify the clinician by using a device, such as a bar code reader, to read the clinicians' badge 116a. The system may also use a biometrics to positively identify the clinician 116, to assure the clinician is an authorized user of the system, and to determine whether the clinician 1176 has authority to access portions of the system 100. The system 100 may require a combination of the clinician badge 116a, or other key, and a verified biometric match in order to grant the clinician access to the system 100. The system may also be configured to terminate access to the system 100 when the clinician badge 116a is removed from the vicinity of the device used to read the clinician badge 116a, or other key.

Biometrics is the technology and science of statistically analyzing measured biological data. One field of biometrics is that of determining unique physical characteristic, such as fingerprints. Biometrics makes it possible to identify individuals to digital systems, such as system 100. A digital persona is created that makes transactions and interactions more convenient and secure. Biometric features for identification include features such as, but not limited to, fingerprint, face, iris and retina scanning, and voice identification. Biometric devices include a scanning or reading device, software to convert the scanned information into a digital format, and a memory to store the biometric information for comparison with a stored record. Software identifies specific matched points of data that have been processed with an algorithm and compares the data. Unlike passwords, PIN codes, and smartcards, the system 100 biometrics cannot be lost, forgotten, or stolen.

The biometric scanner may be associated with the device for reading the clinician's badge 116a. For example, the biometric scanner may be a thumb print reader on the handle of a bar code reader. In other embodiments, the biometric scanner and an electronic key reader may be located on the portable medicine cart and/or the medical device. When the clinician 116 places the electronic key within a specified distance of the medical device, a processor will know the specific individual electronic biometric identification file it should expect. The system 100 preferably prompts the clinician 116 to scan their biometric information. The biometric information is entered into the system 100 with some type of biometric reading or scanning device. A one-to-one comparison is made between the scanned biometric information and the previously stored specific individual electronic biometric identification file. This one-to-one identity comparison is more efficient than comparing one-to-many identity files because it does not require searching an entire clinician database for a match. Instead, only one specific comparison is made. If there is a match, then the clinician 116 is granted access to the medical device 332. If there is no match, the clinician 116 is denied access.

In another embodiment, after the system 100 grants access to the clinician 116, the system 100 may terminate that access when the electronic key is removed from the biometric scanner, or the vicinity of the biometric scanner. The vicinity within which the electronic key must be kept may be predetermined and/or may be a variable and programmable system 100 parameter.

In one embodiment, block 404 includes an encrypted digital fingerprint template, a clinician's name, a login name, and a password. One technology for implementing the clinician identifier includes "IBUTTON 400" technology from Dallas Semiconductor technology. The system 100 may be activated when the clinician places a finger on a fingerprint scanner. If the system 100 finds a match, the system 100 may request the clinician 116 login to the system 100. If the system 100 does not find a biometric match, the system does not allow the clinician 116 to access the system 100.

In another embodiment, the database storing biometric information may be kept in the central system 108, the pharmacy computer 104, and/or the treatment location 106. At the treatment location 106, the database may be maintained in the portable cart, the digital assistant 118, and/or the medical device 332. Such distributed databases will allow access to remote devices even of the network 102 is unable to communicate between the various locations. When network 102 communication is reestablished, the remote and central databases may be synchronized with any information modified at the other location so that both system 100 databases are properly updated.

The system 100 provides a closed loop infusion therapy management system. The closed loop begins with a clinician 116 order. Among other methods, the clinician 116 may enter the order through digital assistant 118 and/or the medical treatment cart 132. After the order is entered, the order is available in real-time for pharmacy authorization and medical screening. The order is available in real-time as an electronic medication administration record (MAR). The MAR is available to the clinician 116 for infusion administration. The system 100 automatically documents infusion administration and modifications such as flow rate changes. Through the process of infusion administration, the system 100 simultaneously adjusts system 100 and/or subsystem inventory. The system 100 also provides data for billing. All phases of the order may be handled in one system 100 in a real-time. The system 100 also provides event management and decision support data. The system 100 is device independent, meaning that it can be run on workstations, wireless tablets and handheld digital assistants 100.

The closed loop infusion therapy management system includes infusion order entry, admixture order preparation, and the availability of the status of the infusion. Infusion order entry may be through a number of systems such as, but not limited to the prescription entry module, the prescription modification module 336, and the pharmacy interface 316. The status of the infusion provides patient 112 specific usage of infusions and alerts the pharmacy of the need for additional infusion bags.

Infusion orders may be entered according to a number of characteristics, single dosing, load dosing, intermittent dosing, and continuous infusions. Continuous infusions include continuous infusions, alternating infusions, sequencing infusions, tapering infusions, and titrating infusions.

Dosing for infusion order entry may be calculated according to body weight or body surface area. Dosing can also be entered according to rate. When the rate is not entered. The system 100 will calculate the rate according to the dose and time period specified. The clinician 116 may specify the diluent and its quantity. The pharmacy may provide a default for such parameters. A system 100 check may be performed to ensure the net concentration for the admixtures and the rate of infusion are appropriate. The system 100 can include system 100 wide defined stop orders. Changes in patient status may generate messages for system 100 defined appropriate action. The system 100 coordinates with the ADT interface 310 to automatically stop orders upon discharge or death.

When admixture orders are entered into the system 100, preparation instructions are routed to a preparation location. The preparation location depends upon the system's100 admixture program and the type of admixture. The system 100 may include adjustable defaults that specify where the admixture is to be filled. The admixture may be filled in the pharmacy or in a remote location, such as on the floor or at the treatment location 106. The clinician 116 is guided through the preparation process using event management information that may be displayed on digital assistant 118. The medication label 124a for the admixture lists the ingredients and respective doses. The medication label 124a may be printed in any location. Upon administering the admixture infusion, the medication label 124 is scanned. Any adjustments made to the flow rate are tracked through using bar code scanning. The pharmacy is alerted in real time to adjust the timing of the next required admixture infusion. Monitoring of admixture preparation and administration allows for a just-in-time delivery of medications 124. This reduces wastage attributed to discontinued or changed preparations. This also ensures patient 112 safety.

The system 100 calculates infusion flow rates based on the patient's weight, body surface area, and/or a specified frequency and duration of therapy. The ordered infusion rate is verified against the maximum push rate tolerance. The concentration of the solution can also be verified. During administration and during flow rate, the clinician 116 is alerted on the digital assistant 118 of the infusion rate and associated parameters. Changes in flow rate are communicated in real-time to the pharmacy. The system 100 includes automatic scheduling of solution delivery based on system 100 defined time tolerances.

The completion of an infusion administration may trigger patient billing through the billing interface 312. The billings interface may include an HL7 interface. If patients are to be charged based on infusion order preparation, the system 100 include a crediting process. The crediting process may be triggered when infusions are returned to the pharmacy for waste or re-entry into the pharmacy inventory management system

The system 104 includes automated messaging to appropriate clinicians 116. For example, when a physician enters new orders, messaging appears in the pharmacy to alert the pharmacists that infusion orders require authorization. Likewise, when infusion orders are appropriately authorized, the clinician 116 receives messaging on digital assistant 118 to alert the clinician 116 that the infusion order should be administered according with in the time period defined for the order.

It should be emphasized that the above-described embodiments of the present invention, particularly, any embodiments, are possible example of implementations, merely set forth for a clear understanding of the principles of the invention. Many variations and modifications may be made to the above-described embodiment(6) of the invention without substantially departing from the scope of the invention. All such modifications are intended to be included herein within the scope of this disclosure and the present invention and protected by the following claims.

## Claims

1. A method for verifying that a medical treatment to be administered to a patient is correct, the method including the use of a system including a first computer and a second computer and comprising the steps of:
accessing information related to the identity of the patient from the second computer at a remote location, said remote location being any location where the patient is receiving treatment ;
accessing information regarding the identity of the medical treatment from the second computer, the medical treatment having a treatment type;
determining whether the medical treatment has been previously associated with the patient:
providing a first error signal if the medical treatment has not teen previously associated with the patient;
accessing information related to the identity of a medical device from the second computer, the medical device configured to administer the medical treatment type;
determining whether a plurality of operating parameters including a flow rate for the medical device are consistent with the medical treatment, the operating parameters having been provided from the first computer at a central location, said central location being any location other than the remote location where parameters for operating the medical device are accessible, the operating parameters having been provided to the medical device without passing through the second computer and
providing a second error signal if the operating parameters for the medical device are not consistent with the medical treatment; enabling a nurse or a clinician to adjust the flow rate within a designated flow rate adjustment tolerance associated with a medication being provided by the medical device, wherein the system stores a predefined indication regarding whether the nurse or clinician is permitted to override the flow rate adjustment tolerance; and if the nurse or clinician if permitted to override the flow rate adjustment tolerance, enabling the nurse or clinician to override the flow rate adjustment tolerance.

2. The method of Claim 1. where the medical treatment has been previously associated with the patient by providing the first computer with a patient identifier and a medication identifier, where the medication identifier includes the patient identifier.

3. The method of Claim 2, where the step of accessing information regarding 5. the identity of a medical treatment includes inputting into the second computer a second medication identifier, where the second medication identifier includes the patient

4. The method of Claim 3, where the step of providing operating parameters for the medical device is performed only if the first and second medication identifiers include the same patient identifier.

5. The method of Claim 1, where the medical device is an infusion pump.

6. The method of Claim 1, where providing the first computer with a medication Identifier includes converting a signal generated by an input device to a computer readable medium format.

7. The method of Claim 1, where the patient identifier is one of a group of identifiers, where the group of identifiers consists of: a patient name, a patient social security number, a patient blood type, a patient address, a patient's allergy, a hospital patient ID number, a hospital bed location, and a name of a patient's relative.

8. The method of Claim 1, where the operating parameter is one of a group of operating parameters, where the group of operating parameters consists of a medication flow per unit of time, a quantity of medication, a dosing unit, a dosing duration, a dosing volume, a drug name, a dose unit, and a monitoring limit.

9. The method of Claim 1, including a step of inputting into the second computer from a first source a signal generated by an input device and converted to a computer readable medium format.

10. The method of Claim 1, where the step idenfifying whether the clinician is permitted to override the flow rate adjustment tolerance includes accessing information related to the identity of a clinician by using a bar code scanner to read the clinician's badge, and the operating parameters having been provided to the medical device using a wireless communication path.

11. A method for programming a medical device to administer a medical treatment, the method including the use of a system including a first computer and a second computer and comprising the steps of:
accessing information related to the identity of a patient from the second computer at a remote location, said remote location being any
location where the patient is receiving treatment ;
accessing information regarding the identity of the medical treatment from the second computer, the medical treatment having a treatment type;
determining whether the medical treatment has been previously associated with the patient;
providing a first error signal if the medical treatment has not been previously associated with the patient.
accessing information related to the identity of the medical device from the second computer, the medical device configured to administer the medical treatment type;
determining whether a plurality of operating parameters including a flow rate for the medical device are consistent with the medical treatment, the operating parameters having been provided from the first computer at a central location, said central location being any location other than the remote location where parameters for operating the medical device are accessible the operating parameters having been provided to the medical device without passing through the second computer;
providing a second error signal it the operating parameters for the medical device are not consistent with the medical treatment;
enabling a nurse or a clinician to adjust the flow rate within a designated flow rate adjustment tolerance associated with a medication being provided by the medical device, wherein the system stores a predefined indication regarding whether the nurse or clinician is permitted to override the flow rate adjustment tolerance; and
if the nurse or clinician is permitted to override the flow rate adjustment tolerance, enabling the nurse or clinician to override the flow rate adjustment tolerance.

## Patentansprüche

1. Verfahren zum Überprüfen, ob eine an einen Patienten zu verabreichende medizinische Behandlung richtig ist, wobei das Verfahren die Verwendung eines Systems, enthaltend einen ersten Computer und einen zweiten Computer, beinhaltet und die Schritte umfasst:
Abrufen von Information in Bezug auf die Identität des Patienten von dem zweiten Computer an einem entfernten Ort, wobei der entfernte Ort jeglicher Ort ist, an dem der Patient die Behandlung erhält,
Abrufen von Information in Bezug auf die Identität der medizinischen Behandlung von dem zweiten Computer, wobei die medizinische Behandlung einen Behandlungstypus aufweist,
Bestimmen, ob die medizinische Behandlung früher mit dem Patienten in Zusammenhang stand,
Bereitstellen einer ersten Fehlermeldung, wenn die medizinische Behandlung nicht früher mit dem Patienten in Zusammenhang stand,
Abrufen von Information in Bezug auf die Identität einer medizinischen Vorrichtung von dem zweiten Computer, wobei die medizinische Vorrichtung konfiguriert ist, den medizinischen Behandlungstypus zu verabreichen, Bestimmen, ob eine Mehrzahl von Betriebsparametern, einschließlich einer Flussrate für die medizinische Vorrichtung, mit der medizinischen Behandlung übereinstimmen, wobei die Betriebsparameter von dem ersten Computer an einem zentralen Ort bereitgestellt wurden, wobei der zentrale Ort jeglicher Ort mit Ausnahme des entfernten Orts ist, an dem Parameter für den Betrieb der medizinischen Vorrichtung zugänglich sind, wobei die Betriebsparameter in der medizinischen Vorrichtung bereitgestellt wurden, ohne durch den zweiten Computer geleitet zu werden, und
Bereitstellen einer zweiten Fehlermeldung, wenn die Betriebsparameter für die medizinische Vorrichtung nicht mit der medizinischen Behandlung übereinstimmen,
Ermöglichen einem Krankenpfleger/einer Krankenpflegerin oder einem Kliniker/einer Klinikerin, die Flussrate innerhalb einer vorgesehenen Toleranz für die Anpassung der Flussrate, welche mit einer medikamentösen Behandlung, die von der medizinischen Vorrichtung bereitgestellt wird, in Zusammenhang steht, anzupassen, wobei das System eine vorher festgelegte Angabe speichert, ob es dem Krankenpfleger/der Krankenpflegerin oder dem Kliniker/der Klinikerin erlaubt ist, sich über die Toleranz für die Anpassung der Flussrate hinwegzusetzen, und
wenn es dem Krankenpfleger/der Krankenpflegerin oder dem Kliniker/der Klinikerin erlaubt ist, sich über die Toleranz für die Anpassung der Flussrate hinwegzusetzen, Ermöglichen dem Krankenpfleger/der Krankenpflegerin oder dem Kliniker/der Klinikerin, sich über die Toleranz für die Anpassung der Flussrate hinwegzusetzen.

2. Verfahren nach Anspruch 1, wobei die medizinischen Behandlung zuvor durch Bereitstellen einer Patientenkennung und einer Kennung der medikamentösen Behandlung in dem ersten Computer mit dem Patienten in Zusammenhang gebracht wurde, wobei die Kennung der medikamentösen Behandlung die Patientenkennung enthält.

3. Verfahren nach Anspruch 2, wobei der Schritt des Abrufens von Information in Bezug auf die Identität einer medizinischen Behandlung das Eingeben einer zweiten Kennung der medikamentösen Behandlung in den zweiten Computer beinhaltet, wobei die zweite Kennung der medikamentösen Behandlung die Patientenkennung enthält.

4. Verfahren nach Anspruch 3, wobei der Schritt des Bereitstellens von Betriebsparametern für die medizinische Vorrichtung nur durchgeführt wird, wenn die erste und zweite Kennung der medikamentösen Behandlung die gleiche Patientenkennung enthält.

5. Verfahren nach Anspruch 1, wobei die medizinische Vorrichtung eine Infusionspumpe ist.

6. Verfahren nach Anspruch 1, wobei das Bereitstellen einer Kennung der medikamentösen Behandlung in dem ersten Computer das Umwandeln eines Signals, das von einer Eingabevorrichtung erzeugt wurde, in das Format eines von einem Computer lesbaren Mediums beinhaltet.

7. Verfahren nach Anspruch 1, wobei die Patientenkennung eine aus einer Gruppe von Kennungen ist, wobei die Gruppe von Kennungen aus einem Namen des Patienten, einer Sozialversicherungsnummer des Patienten, einer Blutgruppe des Patienten, einer Adresse des Patienten, einer Allergie des Patienten, einer Krankenhauskennnummer des Patienten, einem Ort des Krankenhausbettes und einem Namen eines Verwandten des Patienten besteht.

8. Verfahren nach Anspruch 1, wobei der Betriebsparameter einer aus einer Gruppe von Betriebsparametern ist, wobei die Gruppe von Betriebsparametern aus einem Fluss der medikamentösen Behandlung pro Zeiteinheit, einer Menge der medikamentösen Behandlung, einer Dosierungseinheit, einer Dosierungsdauer, einem Dosierungsvolumen, einem Arzneimittelnamen, einer Dosiseinheit und einem Überwachungsgrenzwert besteht.

9. Verfahren nach Anspruch 1, enthaltend einen Schritt des Eingebens eines Signals, das von einer Eingabevorrichtung erzeugt und in das Format eines von einem Computer lesbaren Mediums umgewandelt wurde, aus einer ersten Quelle in den zweiten Computer.

10. Verfahren nach Anspruch 1, wobei der Schritt des Ermittelns, ob es dem Kliniker/der Klinikerin erlaubt ist, sich über die Toleranz für die Anpassung der Flussrate hinwegzusetzen, das Abrufen von Information in Bezug auf die Identität des Klinikers/der Klinikerin durch Verwendung eines Strichcodelesers, um das Erkennungszeichen des Klinikers/der Klinikerin zu lesen, beinhaltet, und die Betriebsparameter in der medizinischen Vorrichtung unter Verwendung eines drahtlosen Kommunikationswegs bereitgestellt wurden.

11. Verfahren zur Programmierung einer medizinischen Vorrichtung, um eine medizinische Behandlung zu verabreichen, wobei das Verfahren die Verwendung eines Systems, enthaltend einen ersten Computer und einen zweiten Computer, beinhaltet und die Schritte umfasst:
Abrufen von Information in Bezug auf die Identität eines Patienten von dem zweiten Computer an einem entfernten Ort, wobei der entfernte Ort jeglicher Ort ist, an dem der Patient die Behandlung erhält,
Abrufen von Information in Bezug auf die Identität der medizinischen Behandlung von dem zweiten Computer, wobei die medizinische Behandlung einen Behandlungstypus aufweist,
Bestimmen, ob die medizinische Behandlung früher mit dem Patienten in Zusammenhang stand,
Bereitstellen einer ersten Fehlermeldung, wenn die medizinische Behandlung nicht früher mit dem Patienten in Zusammenhang stand,
Abrufen von Information in Bezug auf die Identität der medizinischen Vorrichtung von dem zweiten Computer, wobei die medizinische Vorrichtung konfiguriert ist, den medizinischen Behandlungstypus zu verabreichen, Bestimmen, ob eine Mehrzahl von Betriebsparametern, einschließlich einer Flussrate für die medizinische Vorrichtung, mit der medizinischen Behandlung übereinstimmen, wobei die Betriebsparameter von dem ersten Computer an einem zentralen Ort bereitgestellt wurden, wobei der zentrale Ort jeglicher Ort mit Ausnahme des entfernten Orts ist, an dem Parameter für den Betrieb der medizinischen Vorrichtung zugänglich sind, wobei die Betriebsparameter in der medizinischen Vorrichtung bereitgestellt wurden, ohne durch den zweiten Computer geleitet zu werden,
Bereitstellen einer zweiten Fehlermeldung, wenn die Betriebsparameter für die medizinische Vorrichtung nicht mit der medizinischen Behandlung übereinstimmen,
Ermöglichen einem Krankenpfleger/einer Krankenpflegerin oder einem Kliniker/einer Klinikerin, die Flussrate innerhalb einer vorgesehenen Toleranz für die Anpassung der Flussrate, welche mit einer medikamentösen Behandlung, die von der medizinischen Vorrichtung bereitgestellt wird, in Zusammenhang steht, anzupassen, wobei das System eine vorher festgelegte Angabe speichert, ob es dem Krankenpfleger/der Krankenpflegerin oder dem Kliniker/der Klinikerin erlaubt ist, sich über die Toleranz für die Anpassung der Flussrate hinwegzusetzen, und
wenn es dem Krankenpfleger/der Krankenpflegerin oder dem Kliniker/der Klinikerin erlaubt ist, sich über die Toleranz für die Anpassung der Flussrate hinwegzusetzen, Ermöglichen dem Krankenpfleger/der Krankenpflegerin oder dem Kliniker/der Klinikerin, sich über die Toleranz für die Anpassung der Flussrate hinwegzusetzen.

## Revendications

1. Procédé pour vérifier qu'un traitement médical devant être administré à un patient est correct, le procédé comprenant l'utilisation d'un système comprenant un premier ordinateur et un second ordinateur et comprenant les étapes consistant à :
accéder à des informations relatives à l'identité du patient à partir du second ordinateur dans un lieu distant, ledit lieu distant étant un lieu quelconque où le patient reçoit un traitement ;
accéder à des informations concernant l'identité du traitement médical à partir du second ordinateur, le traitement médical ayant un type de traitement ;
déterminer si le traitement médical a été précédemment associé au patient ;
fournir un premier signal d'erreur si le traitement médical n'a pas été précédemment associé au patient ;
accéder à des informations relatives à l'identité d'un dispositif médical à partir du second ordinateur, le dispositif médical étant conçu pour administrer le type de traitement médical ;
déterminer si une pluralité de paramètres de fonctionnement comprenant le débit du dispositif médical sont cohérents avec le traitement médical, les paramètres de fonctionnement ayant été fournis à partir du premier ordinateur dans un lieu central, ledit lieu central étant tout lieu autre que le lieu distant où les paramètres de fonctionnement du dispositif médical sont accessibles, les paramètres de fonctionnement ayant été fournis au dispositif médical sans passer par le second ordinateur ; et
fournir un second signal d'erreur si les paramètres de fonctionnement du dispositif médical ne sont pas cohérents avec le traitement médical ; permettre à une infirmière ou à un médecin de régler le débit dans les limites d'une tolérance donnée de réglage de débit associée à un médicament fourni par le dispositif médical, le système stockant une indication prédéfinie du fait que l'infirmière ou le médecin sont autorisés ou non à corriger la tolérance de réglage du débit ; et si l'infirmière ou le médecin sont autorisés à corriger la tolérance de réglage du débit, permettre à l'infirmière ou au médecin de corriger la tolérance de réglage du débit.

2. Procédé selon la revendication 1, où le traitement médical a été précédemment associé au patient par la fourniture au premier ordinateur d'un identifiant du patient et d'un identifiant du médicament, l'identifiant du médicament comprenant l'identifiant du patient.

3. Procédé selon la revendication 2, où l'étape d'accès aux informations concernant l'identité du traitement médical comprend l'entrée dans le second ordinateur d'un second identifiant de médicament, le second identifiant de médicament comprenant l'identifiant du patient.

4. Procédé selon la revendication 3, où l'étape de fourniture des paramètres de fonctionnement du dispositif médical est effectuée uniquement si les premier et second identifiants de médicament comprennent le même identifiant de patient.

5. Procédé selon la revendication 1, où le dispositif médical est une pompe à perfusion.

6. Procédé selon la revendication 1, où la fourniture au premier ordinateur d'un identifiant de médicament comprend la conversion d'un signal produit par un périphérique d'entrée en un format de support lisible par ordinateur.

7. Procédé selon la revendication 1, où l'identifiant du patient est un élément d'un groupe d'identifiants, ledit groupe d'identifiants étant constitué par : le nom du patient, le numéro de sécurité sociale du patient, le type sanguin du patient, l'adresse du patient, l'allergie du patient, le numéro de dossier d'hospitalisation du patient, l'emplacement du lit d'hôpital, et le nom d'un proche du patient.

8. Procédé selon la revendication 1, où le paramètre de fonctionnement est un élément d'un groupe de paramètres de fonctionnement, ledit groupe de paramètres de fonctionnement étant constitué par : le débit de médicament par unité de temps ; la quantité de médicament, l'unité de dosage, la durée de dosage, le volume de dosage, le nom du médicament, la dose unitaire, et la limite de surveillance.

9. Procédé selon la revendication 1, comprenant une étape consistant à entrer dans le second ordinateur, à partir d'une première source, un signal produit par un périphérique d'entrée et converti en un format de support lisible par ordinateur.

10. Procédé selon la revendication 1, où l'étape établissant si le médecin est autorisé ou non à corriger la tolérance de réglage du débit comprend l'accès aux informations relatives à l'identité d'un médecin au moyen d'un lecteur de code barre permettant de lire le badge du médecin, et les paramètres de fonctionnement ayant été fournis au dispositif médical au moyen d'une voie de communication sans fil.

11. Procédé de programmation d'un dispositif médical pour l'administration d'un traitement médical, le procédé comprenant l'utilisation d'un système comprenant un premier ordinateur et un second ordinateur et comprenant les étapes consistant à :
accéder à des informations relatives à l'identité d'un patient à partir du second ordinateur dans un lieu distant, ledit lieu distant étant un lieu quelconque où le patient reçoit un traitement ;
accéder à des informations concernant l'identité du traitement médical à partir du second ordinateur, le traitement médical ayant un type de traitement ;
déterminer si le traitement médical a été précédemment associé au patient ;
fournir un premier signal d'erreur si le traitement médical n'a pas été précédemment associé au patient ;
accéder à des informations relatives à l'identité du dispositif médical à partir du second ordinateur, le dispositif médical étant conçu pour administrer le type de traitement médical ;
déterminer si une pluralité de paramètres de fonctionnement comprenant le débit du dispositif médical sont cohérents avec le traitement médical, les paramètres de fonctionnement ayant été fournis à partir du premier ordinateur dans un lieu central, ledit lieu central étant tout lieu autre que le lieu distant où les paramètres de fonctionnement du dispositif médical sont accessibles, les paramètres de fonctionnement ayant été fournis au dispositif médical sans passer par le second ordinateur ;
fournir un second signal d'erreur si les paramètres de fonctionnement du dispositif médical ne sont pas cohérents avec le traitement médical ;
permettre à une infirmière ou à un médecin de régler le débit dans les limites d'une tolérance donnée de réglage de débit associée à un médicament fourni par le dispositif médical, le système stockant une indication prédéfinie du fait que l'infirmière ou le médecin sont autorisés ou non à corriger la tolérance de réglage du débit ; et
si l'infirmière ou le médecin sont autorisés à corriger la tolérance de réglage du débit, permettre à l'infirmière ou au médecin de corriger la tolérance de réglage du débit.
